Europäisches Patentamt

European Patent Office

Office européen des brevets

(19)

(11) EP 0 730 146 A2

(12) EUROPEAN PATENT APPLICATION

(43) Date of publication:
04.09.1996 Bulletin 1996/36

(51) Int Cl.$^6$: G01N 21/84

(21) Application number: 96301383.4

(22) Date of filing: 29.02.1996

(84) Designated Contracting States:
DE FR GB IT NL

(30) Priority: 01.03.1995 DK 212/95

(71) Applicant: Slagteriernes Forskningsinstitut
DK-4000 Roskilde (DK)

(72) Inventors:
• Thodberg, Hans Henrik
4000 Roskilde (DK)

• Madsen, Niels T.
2670 Greve (DK)

(74) Representative: Bayliss, Geoffrey Cyril et al
BOULT, WADE & TENNANT
27 Furnival Street
London EC4A 1PQ (GB)

(54) Method for the determination of quality properties of individual carcasses, lamp for the illumination of a carcass and use of the method and lamp

(57) Quality properties of individual carcasses are determined in a production room by recording of a first picture of a carcass by means of a colour video camera and registration of the picture. The carcass is illuminated by lamps (5) which provide an illumination of the carcass which is one or several times the illumination of the carcass from the surroundings. A second picture of the carcass is recorded without any other illumination than the illumination of the carcass from the surroundings. The R,G,B-light values for the pixels in the non-illuminated recording are subtracted from the R,G,B-light values for the corresponding pixels in the illuminated recording. The resulting R,G,B-light values are used for data processing and analysis according to a predetermined procedure in the determination of the quality properties of the carcass.

During the recordings the carcass is placed in front of an opaque plate (6) which has a larger dimension than the carcass. It is mainly unscreened against light coming from the surroundings, as this is eliminated by means of the second recording and the subsequent subtraction.

A lamp for the illumination of a carcass during a recording is placed comparatively close to the carcass, i. e. at a distance which is smaller than the length of the carcass. The lamp includes an oblong housing in which several light sources are mounted The light sources are placed closer to each other at the end areas of the housing than at the centre of the housing, and they are preferably placed progressively closer to each other towards the ends of the housing. The lamp produces a uniform illumination of the carcass.

The method and the lamp are preferably used for automatic classification of carcasses, particularly cattle carcasses.

FIG. 1

## Description

The present invention relates to a method for the determination of quality properties of individual carcasses in a production room, comprising recording of a picture of a carcass which is illuminated by lamps by means of a colour video camera, and registration of the picture, followed by data processing and analysis of the picture according to a predetermined procedure.

Cattle carcasses are today classified visually by skilled graders, who will determine, not later than one hour after slaughter, the class of conformation and fatness of the carcass according to a set of norms (EUROP) existing within the EU. A visual classification of the carcasses according to their colour is also performed as a national Danish arrangement. Classification is carried out in order to give the producers a uniform and fair payment for the animals delivered.

For many years the producers have wanted a classification system which is based upon objectivity and does not require the assistance of a grader. An advantage of such a system is that the valuation of the carcasses will become more constant, and it will also be capable of providing information of the tallow colour of the carcass, and the content of meat, tallow and bones, as well as information of the distribution in the carcass of expensive and cheap cuts. This information can improve the basis for payment, and the slaughterhouse will be able to carry out a more detailed sorting and treatment of the carcasses.

Until now, the most promising systems that have been suggested or tested for objective cattle classification have faced difficulties in becoming accepted.

EP-B-0321.981 (Slagteriernes Forskningsinstitut) describes a system for determination of the EUROP class of conformation and fatness of cattle carcasses. The system determines the contours and tallow cover of the carcasses on the basis of two video pictures recorded in a cabinet. One picture is recorded while the carcass stands out as a dark silhouette against a light-emitting background. The second picture is recorded while the carcass is illuminated by lamps on the side facing the video camera. The purpose of the cabinet is to screen off foreign light, to reduce the amount of indirect light, and to protect the operators working close to the system from the inconvenience of the lamps.

It has not been possible for all slaughterhouses to find space for placing the required cabinet. Thus, the system has not been able to comply with the need for a common standard for classification in Danish cattle slaughterhouses.

WO-A-91/14180 (Australian Meat & Livestock Research & Development Corp.) describes a system for classification of carcasses according to shape. It includes a video camera and a lamp which is placed on top of the camera. In the field of vision of the camera is a dark plate which serves as background at the recordings. At first a picture of the plate only is recorded. Then a carcass is placed in front of the plate, and a picture of the carcass and the plate together is recorded. In this way the background can be eliminated from the recording with the carcass, so that the contour of the carcass will appear as a sharp picture, even if the plate becomes polluted during the daily operation. It is mentioned in said description that the system can be installed on slaughterlines and that no cabinet will be required. However, without screening against foreign light the carcasses cannot be classified according to shape in a reliable way, and a determination of the colour of tallow and meat is not possible without a cabinet.

A possible alternative enabling removal of the cabinet is to use a very powerful illumination, so that the light of the surroundings becomes negligible. By exposing the carcass to an illumination which is e.g. 40 times more powerful than the illumination of the surroundings, the error in the determination of the light values of the video picture will be 2.5% at most, and this may be acceptable. However, the method demands considerable resources, and the indirect light from the powerful light sources may cause problems that will require the use of special lamps. To the operators in the room the illumination will moreover be a significant inconvenience. The disadvantages can be reduced somewhat by moving the lamp as close to the carcass as possible and by removing surfaces that may cause indirect light, or by painting them in a dark colour. Much consideration will be required at the installation and maintenance.

An object of the present invention is to provide a method which renders the use of a cabinet superfluous and enables the use of lamps which do not inconvenience the operators working in the room. Hereby, the measuring results must be relatively unaffected by the light conditions of the surroundings.

The method of the present invention is characterized in that the recording of the picture is made with an illumination of the carcass which is one or several times the illumination of the carcass from the surroundings, that a second picture of the carcass is recorded without any other illumination than the illumination of the carcass from the surroundings, and that the R,G,B-light values for the pixels in the non-illuminated recording are subtracted from the R,G,B-light values for the corresponding pixels in the illuminated recording, the resulting R,G,B-light values then being used in the data processing and analysis for the determination of quality properties of the carcass.

Although the illumination used in the method of the invention is more powerful than the illumination from the surroundings it is nevertheless possible to keep it at a low level, so that the environmental inconveniences and other disadvantages are only insignificant. There is no requirement that the illumination must be a large multiple of the illumination from the surroundings. The method of the invention does already work at relatively low light intensity from the lamps.

The method of the invention makes no use of a light-screening cabinet. During the recording with switched-on lamps the carcass is not only illuminated by the lamps, but also by the background light from the surroundings, i.e. mainly the production room. In order to reduce or remove the effect of the background light, one more picture is recorded, but with the lamps switched off, and the two pictures are subtracted pixel by pixel. The differential picture created mainly corresponds to what would be recorded if the carcass was illuminated by the lamps only.

The explanation why it is possible to reduce or eliminate the background light is that the intensity, I, of the light from a carcass which is illuminated by several light sources is equal to the sum of the intensities which exist by illumination of the carcass by the sources individually. If I(L+B) is the intensity of the light falling on an element of the light-sensitive plate of the video camera (e.g. a CCD-chip) from an area of the carcass, while it is being illuminated by a light source, L, and other light sources in the form of background light, B, the following equation applies:

$$I(L+B) = I(L) + I(B)$$

in which I(L) is the intensity from the light source, L, only, and I(B) is the intensity from the background light, B, only. In other words, I(L) - which also expresses the true value of the relative light-reflective power of said area - can be determined when I(L+B) and I(B) are known.

It is a prerequisite for the equation that there is linearity, i.e. that the value of intensity measured by an element of the light-sensitive plate of the camera is proportional with the light intensity on the element. This is normally not the case in the common video cameras. To achieve linearity it is necessary to use a camera with adjustable gamma correction and to make the necessary adjustments and modifications. However, even if liearity is not established or achieved, the method of the invention will reduce the effect of the background light, but to at lesser degree, the above equation becoming an approximation.

By recording one picture with illumination and another picture without illumination of the carcass, and subtracting the pictures from each other, it is possible to obtain the same advantages as those obtained until now by use of a cabinet:

- foreign light is eliminated or reduced,
- indirect light from lamps can be kept at a constant level by placing the lamps close to the carcass and/ or by using focusing or screened lamps,
- good light environment in the room.

An embodiment of the method of the invention is characterized in that the carcass during the recordings is placed in front of an opaque plate having a dimension which is larger than the carcass (and preferably taking up the entire field of vision of the camera), and that the carcass during the recordings is mainly unscreened against light coming from the surroundings. By using this minimum screening irrelevant objects in the room are excluded from the recordings, and the plate prevents the operators from being inconvenienced by the lamps. In special situations the carcass may be screened from particularly strong light sources, such as the sun or flashing-type light sources, e.g. hazard flashers.

In the method of the invention the pictures are preferably recorded immediately one after the other, preferably at intervals of a second or less. By recording the two pictures quickly after each other, there is not time for the character and intensity of the background light to change between the pictures.

Therefore, flashlight can be used for illumination of the carcass. The illumination should preferably be diffuse. An advantage achieved by using flashlight is that there is no need for the carcass to be still during the recording process.

However, a drawback by using common video cameras is that they only record one half-frame during the short period that a flashlight is active. The information from the other half-frame is lost, and the error in the determination of the curvature of the thigh of a carcass will consequently become twice as large as otherwise, since the carcass is recorded in portrait format. An exact determination of the curvature is important for the correct determination of the class of conformation of the carcass.

In order to get as much information as possible, the video cameras used are therefore preferably designed to record a full-frame within one flashlight period. Another possibility is to flash twice in rapid succession and record one half-frame in each flashlight period.

When using common video cameras the lamps preferably emit light while both half-frames of the illuminated carcass are being recorded (not flashlight).

The carcass preferably assumes the same position of rest during the illuminated and the non-illuminated recording, but it is also acceptable that the carcass is not at rest, e.g. when the amplitudes are not too big, e.g. less than one cm, or the two pictures are adjusted after each other, so that the carcass is located at the same place in the pictures.

The illumination of the carcass by the lamps is preferably between 1 and 15 times the illumination of the carcass from the surroundings. The effect of the background light can thereby be eliminated without any visual effect on the differential picture, and besides, the illumination is not so strong that it will inconvenience the operators. It is particularly suitable to use an illumination of the carcass which is between 2 and 6 times the illumination of the carcass from the surroundings, since this illumination will be acceptable to the operators as a matter of course.

The intensity of illumination in production rooms used for cattle classification is normally between 50 and 200 lux, and it may locally be as high as 300 lux. In order to ensure a good margin and achieve a suitably low noise level in the differential picture, the illumination intensity of the lamps on the carcass is preferably at least 500 lux.

On the other hand, the illumination of the carcass from the lamps should be 2,000 lux at most, in order not to inconvenience the operators.

Preferably, the pictures are digitized before they are subtracted. In this way the pictures can quickly be stored in an electronic memory and subsequently be subtracted from each other, pixel by pixel. A so-called framegrabber can be used for the digitizing and storage of the pictures.

The light intensity and colour temperature of the lamps may change during the operation of the apparatus. Likewise, the sensitivity of the camera may vary. In order to counteract this instability, colour pads can be placed in the field of vision of the camera and be used for adjustment of the camera, lamps and/or the R,G,B-light values of the pictures.

It appears from the above that the purpose of the invention is served in carcassicular by use of lamps which are placed fairly close to the carcass. By this, the lamps need not be very powerful, i.e. the environmental inconvenience is minimized, and the indirect light from the lamps coming from uncontrollable, reflective surfaces (floor and walls) is kept at a minimum. However, this makes special demands on the lamps, as the illumination of the carcass must be uniform all over the side that is facing the camera. Otherwise, the dynamic range of the camera will not be utilized at its optimum, and the interpretation of the differential picture is made more difficult.

Therefore, the invention also relates to a lamp which can be placed comparatively close to a carcass providing a uniform illumination.

More specifically, the invention relates to a lamp for the illumination of a carcass during recording of a colour video picture of the carcass with an illumination which is one or several times the illumination of the carcass from the surroundings, the carcass being positioned at a distance to the lamp which is smaller than the length of the carcass, and the lamp is characterized in that it comprises an oblong house, in which are mounted several light sources which are placed closer to each other at the end areas of the house than at the centre of the house. By this, a uniform illumination of the carcasses is achieved.

The lamp of the invention meets the demands for a uniform illumination of cattle carcasses which are to be classified by means of video recordings. Due to the preferred relatively small dimensions of the lamp, it can be accommodated in slaughterhouses.

Ideally, a lamp used for illumination of a carcass should be infinitely long in order to provide a uniform illumination of the carcass. However, because of the special positioning of the light sources in the lamp of the invention, approximately the same effect is produced with a lamp that is considerably shorter. This means that the lamp can be placed in a production room.

Preferably, the lamp of the invention has a housing, the length of which is greater than the length of the carcass, preferably between 1.2 and 1.4 times the length of the carcass. On the one hand, an even illumination of the carcass may be provided in this way, and on the other hand, the physical size of the lamp is no obstacle to placing the lamp in production rooms.

If pictures of only a section of the carcass are to be recorded, by the term length of the carcass is to be understood the length of this section of the carcass only.

A particularly uniform illumination of the carcass is achieved when the light sources at the end areas of the housing are placed at least twice as close to each other as they are in the central area. A particularly advantageous embodiment of the lamp of the invention is characterised in that the light sources located at the end areas of the housing are positioned progressively closer to each other towards the ends of the housing, preferably by reducing the distance between the light sources by a factor between 0.6 and 0.9 towards the ends of the housing.

The light sources are preferably point or spherical light sources, for example, carcassicular halogen bulbs.

The requirements for illumination of a cattle carcass which is to be classified on the basis of video recordings are best met by halogen lamps. The spectrum from these lamps is even and without lines, and it is also so wide that it covers the entire visible range. The lamps fulfil a general wish that the colours can be recorded with the same correctness as in daylight (with the camera adjustment of the white balance).

This is a significant advantage in comparison with fluorescent tubes which have distinct peaks in the spectrum. Furthermore, halogen lamps can be switched on quickly and they have a long life even if they are switched on repeatedly. They achieve 90% of their maximum light intensity in approx. 0.1 second, whereas fluorescent tubes require approx. 100 seconds. Besides, halogen lamps are easy to position as desired in the housing of the lamp, and they can be connected direct with the mains. They do not age significantly during the first 1,500 hours of operation and they do not suffer damage from being switched on and off continually.

The housing of the lamp preferably includes an opal plate that is placed in front of the light aperture of the housing, so that the light from the light sources is distributed more uniformly and reflections from lamps are avoided in the video picture.

The plate is preferably of a width that is 1/10 or more of its length. When the area of the light aperture is made comparatively large, the light will inconvenience the operators less.

The lamp preferably contains between 10 and 30 light sources. This modest number of light sources can be placed in such a way that the lamp produces a very uniform illumination.

The method and the lamp of the invention can generally be used in video recordings of various carcasses in production rooms. However, the invention is used with special advantage in video recordings of cattle carcasses, where it is otherwise difficult to consider demands for a light-screening cabinet and voluminous lamps.

The invention also relates to the use of the method and lamp in automatic classification of carcasses, in particular cattle carcasses. By classification is meant determination of the class of conformation or fatness of the carcass according to e.g. the EUROP norm and/or determination of other properties that are determined on the basis of the surface condition of the carcasses, e.g. the colour of meat or tallow.

In the present description and the claims : by the term carcass is meant a whole carcass, a carcass half (split carcass) and/or a carcass quarter.

By way of example, a specific embodiment in accordance with the invention will be described with reference to the accompanying drawings, in which :

Fig. 1 shows an apparatus for automatic classification of cattle carcasses,
Fig. 2 the apparatus seen from above,
Fig. 3 a lamp used in the apparatus,
Fig. 4 the illumination intensity of the lamp at different positions of its bulbs, and
Fig. 5 the illumination intensity of the lamp at different distances.

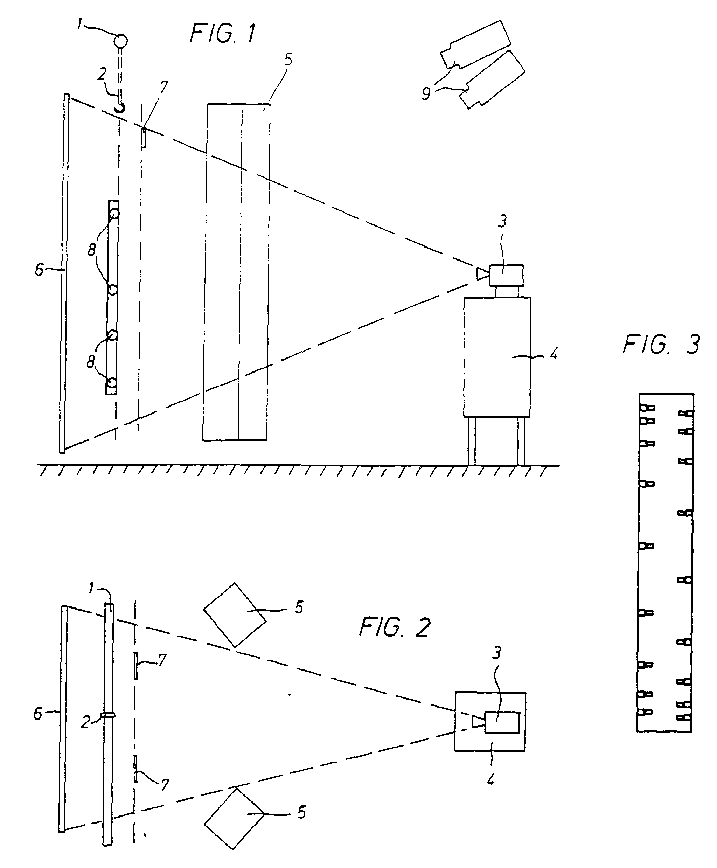

The conveyor 1 (Fig. 1) of a slaughter line conveys half (split) cattle carcasses, each suspended in a gambrel 2. The apparatus for classification of carcasses is placed by the conveyor. It comprises a 3-chip CCD-colour video camera 3, which is laid on the side, so that the pictures of a carcass half being conveyed into the field of vision is recorded in portrait format (upright position). The camera is connected with a data unit 4 which contains various electronics for controlling the measuring procedure during the recording of video pictures, and electronic circuits and programs for storage and processing of the pictures. Furthermore, the data unit contains programs for interpretation of the pictures with a view to determining the class of conformation and fatness of the carcass and other properties.

The data unit is connected with a monitor and a keyboard used by an operator to key in necessary data, start testing and adjustment procedures, or call up various information. The data unit also has an input for a signal from an electronic weighing machine, which automatically provides information of the weight of the current carcass and slaughter serial number. Furthermore, the unit has an output for a printer which after each measuring procedure will print out a ticket that an operator can attach to the carcass.

The monitor, keyboard and printer can be placed close to the conveyor, so that the operator can easily attach the tickets to the carcasses after the classification, and furthermore make an after-check, if necessary.

Placed in front of the camera 3, but outside of its field of vision, are two lamps 5, the centre of which is on a level with the camera. The lamps form an angle of 45-60° with the optical axis of the camera and illuminate the carcass half from either side. This produces an even illumination of the sides of the carcass, and reflections are prevented. The lamps are placed approx. 1 m from the place where the carcass is located during the recording, i.e. comparatively close to the carcass, and since the camera is furthermore of the sensitive CCD-type, low-power lamps are used which do not cause inconvenience to the operators in the room.

It is important that all parts of the carcass are exposed to the same intensity of light, and this is obtained by making the lamps somewhat taller than the carcass, and by placing 18 units of 75W halogen bulbs in the housing of each lamp in the way shown in Fig. 3. The sockets of the bulbs are placed alternately in one side and in the other side of the housing, 20 cm behind the front of the lamp. Each of the lamps is 290 cm tall, 48 cm wide, and 35 cm deep, and is provided with an opal front plate. The inside of the housing is painted white.

In the field of vision of the camera is placed a matt, green surface or plate 6, which in this embodiment serves as an opaque background for the carcass during the recording of pictures, and which also prevents, to a considerable extent, the light from the lamps from inconveniencing the operators in the room.

At the top of the field of vision of the camera there are two sets of coloured pads 7 which are used for adjustment purposes.

A fixture in the form of some bars 8 has the purpose of pushing the carcass a little in the direction of the camera 3 and holding the carcass half mainly at right angles to the optical axis of the camera during the recording of the pictures. The fixture positions the split-surface of the carcass mainly in the vertical plane which contains the conveyor 1, so that the centre of gravity of the carcass is located in front of the vertical plane. In its drawn-back position the fixture is close to the plate 6, so that a carcass half can unimpededly pass into or out of the field of vision of the camera.

The apparatus may comprise one or several devices for illumination of the carcass in a pattern of light from a direction that is different from the optical axis of the camera 5. The devices can for example be two projectors (slide projectors) 9 which form a light raster (line pattern) on a carcass which is placed in the field of vision of the camera. In the slide's place in each projector there has been inserted an opaque plate provided with parallel slits, through which the light from the bulb of the projector can pass. The plate is preferably tilted around an axis which is perpendicular to the plane containing the direction of illumination of the projector and the optical axis of the camera, so that the plate forms a small angle to the optical axis of the camera. The plate is preferably tilted sufficiently that the light from the projector is sharply projected on all parts of the carcass. On the surface of the carcass there is formed a pattern of light which is characteristic of the surface shape of the individual carcass and can be used for the conformation classification of the carcass.

Classification of a carcass by means of the apparatus in Fig. 1 is made by bringing a carcass to a standstill at a certain place on the conveyor 1, after which the fixture is moved forward and positions the split-surface of the carcass mainly at right angles to the optical axis of the camera. The lamps 5 are switched on and a picture of the illuminated carcass is recorded. The picture is digitized and stored in a memory of the data unit 4. The lamps 5 are switched off, and the camera records a second picture of the carcass. This picture is digitized and also stored in a memory. The recordings may also be performed in reverse order. The two stored pictures are then subtracted from each other, so that the effect of the background illumination is eliminated.

The subtraction is made by subtracting the R,G,B-values of a pixel in the non-illuminated picture from the R,G,B-values of the corresponding pixel in the illuminated picture. The table shows an example of the R,G,B-values of a pixel which can be registered by the picture recordings, and the R,G,B-values of the pixel after the subtraction (meat surface).

|  | R | G | B |
|---|---|---|---|
| Illuminated picture | 140 | 103 | 125 |
| Non-illuminated picture | 20 | 18 | 15 |
| Differential picture | 120 | 85 | 110 |

The subtraction process is repeated for all pixels in the two pictures (or at least for the pixels which concern significant surface areas of the carcass). In this way a picture is provided that is cleaned of the effect of the background light and which only expresses the light intensity and colour of the carcass coming from the illumination with the lamps 5, i.e. the relative reflective power of the different surface areas.

In the present arrangement there is used a ratio between the light of the lamps and the background light of 4:1, the lamps producing an illumination intensity of approx. 1,600 lux. As a precaution, the intensity of the background light has been put at 400 lux, as the intensity varies with time, with the location in the production room, and from slaughterhouse to slaughterhouse.

The pictures are digitized and stored with 256 levels of light intensity. The dynamic range of the system is only reduced by 20% compared with the situation where a cabinet is used for screening of the light. This is fully acceptable.

In order to illustrate that the illumination from the lamps does not inconvenience the operators, it can be assumed, by way of example, that the background light comes from a single line of fluorescent tubes located in the ceiling of the room, four metres away from the carcass. They are to produce a background illumination that is 1/4 of the illumination of the carcass coming from the lamps, and therefore they have the same light intensity at a distance of one metre as the lamps (which are placed one metre from the carcass). This means that the light from the lamps 5 at a distance of four metres is not stronger than the light from the fluorescent tubes located in the ceiling of the room. The inconvenience to the operators who are present in the room outside the operational area of the apparatus is therefore not worse than doubling the number of fluorescent tubes that are switched on for a short period.

After the pictures have been subtracted from each other the resulting differential picture is processed in the data unit 4 with a view to determining the class of conformation and fatness of the carcass and possibly other properties. Suitable programs are used for the interpretation of the picture, such as neural networks, principal component analysis, standard algorithms, etc. The results are printed on a ticket which the operator attaches to the carcass. They can also be used in the form of data files or control signals in the further processing of the carcass, e.g. in the cutting process.

If the apparatus also comprises projectors 9 which form a pattern of light on the carcass, a video picture of the carcass with this pattern of light is also recorded by means of the camera 5, and the picture is analyzed for determination of the surface shape of the carcass. It may take place in a separate (third) recording of a picture, but it is also possible to combine this recording and the recording without illumination from the lamps 5 into one recording, as it is possible in the data unit 4, by means of computer programs, to remove the pattern of light from the picture and provide a picture that corresponds to a recording without illumination from the lamps 5 and the projectors 9. The surface shape determined by means of the pattern of light on the individual carcass is used for the determination of the class of conformation of the carcass, since the data unit contains its own interpretation program.

It may be mentioned that the picture with the pattern of light can be adjusted for the effect of the background light in the same way as the picture with illumination from the lamp 5, i.e. by subtracting the light values of the picture without illumination, so that a differential picture is formed.

When the pictures have been recorded, the fixture is automatically pulled back, so that the fixed carcass half is released. It is brought together with the other carcass half and they are conveyed to further processing in the slaughterhouse.

The apparatus can classify 80 or more carcasses per hour, depending upon the rate of the slaughter line and the computer power available for the picture processing and analysis.

## Example

Optimizing of the positions of bulbs in lamps

The positioning of 18 bulbs placed in a line at a distance of 1.20 m from a 220 cm tall surface is optimized, so that the surface is exposed to a uniform illumination. The light from the bulbs is simulated by means of a computer program that changes the interposition of the bulbs until the intensity of the illumination is uniform all over the surface. The positions of the bulbs at the top and at the bottom are reflections around the centre of the lamp, so only the nine top positions are shown. The illumination intensity at a distance of 1.20 metres from the line of bulbs is calculated, and the variance in the illumination intensity, divided by the square of the mean value is defined as the cost function which is to be minimized by moving the bulbs around.

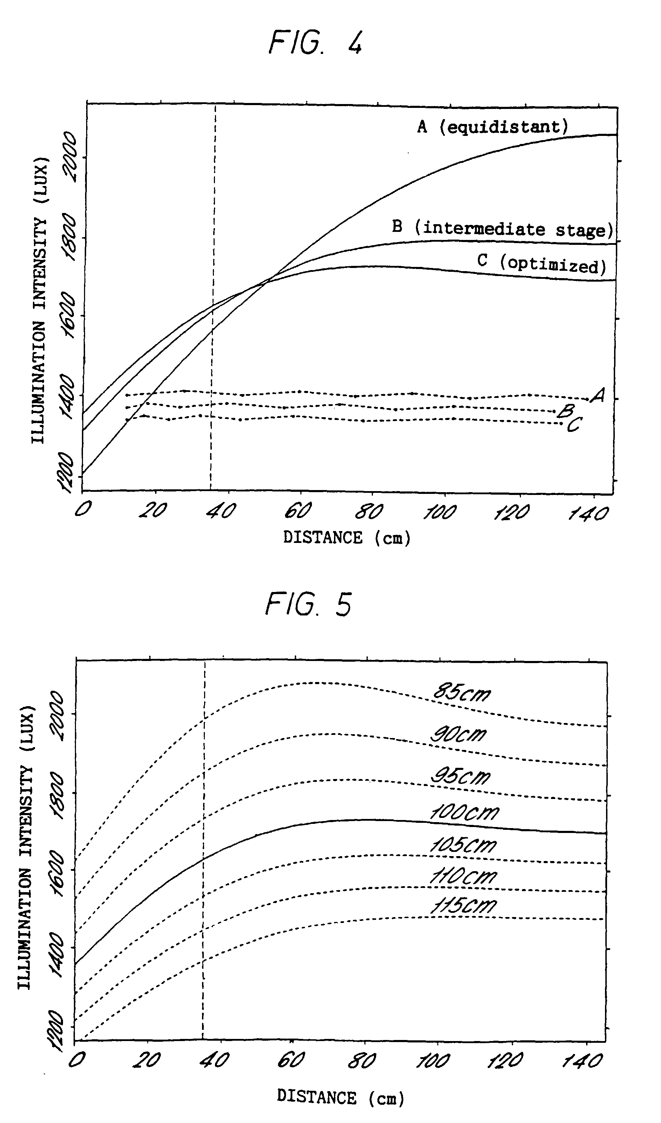

Fig. 4 shows how the optimizing progresses. For a start the bulbs (A) have an equidistant position, which develops via (B) to the optimized solution (C). The position of the bulbs also appears from the figure. It is seen that in the optimized situation the illumination intensity is fairly constant (+/-3%) from 35 cm (the vertical dot-and-dash line) to 145 cm, i.e. over a distance of 110 cm for nine bulbs (or 220 cm as wanted for 18 bulbs). The position (C) corresponds to the one used in the lamp in Fig. 3, where the bulbs are furthermore placed alternately in or on each side of the housing in order to make the light more uniform over the entire width of the carcass.

The illumination intensity can be calculated for other distances. In Fig. 5 there is shown the illumination intensity of the optimized lamp for different distances between the opal plate of the lamp and the surface illuminated (i.e. 20 cm are to be added to obtain the distance to the bulbs). The light is fairly even between 85 and 115 cm from the front of the lamp. Therefore, the lamp is suitable for uniform illumination of the side of a carcass in classification of the carcass by means of video recordings.

## Claims

1. Method for the determination of quality properties of individual carcasses in a production room, comprising recording of a picture of a carcass which is illuminated by lamps by means of a colour video camera, and registration of the picture, followed by data processing and analysis of the picture according to a predetermined procedure, **characterized** in that the recording of the picture is made with an illumination of the carcass which is one or several times the illumination of the carcass from the surroundings, that a second picture of the carcass is recorded without any other illumination than the illumination of the carcass from the surroundings, and that the R,G,B-light values for the pixels in the non-illuminated recording are subtracted from the R,G,B-light values for the corresponding pixels in the illuminated recording, the resulting R,G,B-light values then being used in the data processing and analysis for the determination of quality properties of the carcass.

2. Method according to Claim 1, **characterized** in that the carcass during the recordings is placed in front of an opaque plate of a dimension which is larger than the carcass, and that the carcass during the recordings is mainly unscreened against light coming from the surroundings.

3. Method according to Claim 1 or 2, **characterized** in that the pictures are recorded immediately one after the other, preferably at an interval of one second or less.

4. Method according to any of Claims 1 to 3, **characterized** in that the illumination of the carcass by the lamps is between 1 and 15 times the illumination of the carcass from the surroundings.

5. Lamp for the illumination of a carcass during recording of a colour video picture of the carcass with an illumination which is one or several times the illumination of the carcass from the surroundings, the carcass being positioned at a distance to the lamp which is smaller than the length of the carcass, **characterized** in that the lamp includes an oblong housing in which are mounted several light sources that are placed closer to each other at the end areas of the housing than at the centre of the housing.

6. Lamp according to Claim 5, **characterized** in that the length of the housing is greater than the length of the carcass, preferably between 1.2 and 1.4 times the length of the carcass.

7. Lamp according to Claim 5, **characterized** in that the light sources at the end areas of the housing are positioned progressively closer to each other towards the ends of the housing, preferably by reducing the distance between the light sources by a factor of between 0.6 and 0.9 towards the ends of the housing.

8. Lamp according to Claim 5, **characterized** in that the light sources are point or spherical light sources, preferably halogen bulbs.

9. Lamp according to Claim 5, **characterized** in that it contains between 10 and 30 light sources.

10. Use of the method of Claim 1 and the lamp of Claim 5 in automatic classification of carcasses, preferably cattle carcasses.

EP 0 730 146 A2

FIG. 1

FIG. 3

FIG. 2

8

## FIG. 4

## FIG. 5